Europäisches Patentamt

European Patent Office

Office européen des brevets

(19)

(11) Veröffentlichungsnummer: **0 102 652**
**B1**

(12) # EUROPÄISCHE PATENTSCHRIFT

(45) Veröffentlichungstag der Patentschrift:
08.01.86

(51) Int. Cl.⁴: **C 07 D 213/64** // C07D213/61

(21) Anmeldenummer: 83200700.9

(22) Anmeldetag: 17.05.83

(54) Verfahren zur Reindarstellung von 2-Chlor-6-alkoxy-3-nitropyridinen.

(30) Priorität: 19.08.82 DE 3230828
10.03.83 DE 3308449

(43) Veröffentlichungstag der Anmeldung:
14.03.84 Patentblatt 84/11

(45) Bekanntmachung des Hinweises auf die Patenterteilung:
08.01.86 Patentblatt 86/2

(84) Benannte Vertragsstaaten:
BE CH DE FR GB LI

(56) Entgegenhaltungen:
US - A - 4 310 671

(73) Patentinhaber: **Rütgerswerke Aktiengesellschaft,
Mainzer Landstrasse 217, D-6000 Frankfurt
a.Main 1 (DE)**

(72) Erfinder: **Orth, Winfried, Dr., Am Schachtelgraben 28,
D-6735 Hassloch/Pfalz (DE)**
Erfinder: **Fickert, Werner, Dr., Stockacher Strasse 14,
D-6800 Mannheim (DE)**

## Beschreibung

Die Erfindung betrifft ein Verfahren zur Reindarstellung von 2-Chlor-6-alkoxy-3-nitropyridinen. Diese Verbindungen sind wichtige Zwischenprodukte zur Herstellung von Analgetika.

Aufgrund der pharmazeutischen Verwendung der Endprodukte wird auch bereits bei den Zwischenprodukten eine erhöhte Anforderung an die Reinheit gestellt.

2-Chlor-6-alkoxy-3-nitropyridine werden durch Nitrierung von 2-Chlor-6-alkoxypyridinen mit Salpetersäure dargestellt. Dabei erhält man ein Rohprodukt, das vor der Weiterverarbeitung zu reinigen ist. Üblicherweise erfolgt diese Reinigung durch Umkristallisieren aus Lösungsmittelgemischen. Die unerwünschten Verunreinigungen lassen sich allerdings damit nur schwer abtrennen, so dass vielfaches Umkristallisieren notwendig ist. Abgesehen von dem enormen Arbeits- und Energieaufwand dieses Verfahrens leidet darunter auch die Ausbeute, so dass die Reinprodutke nur in etwa 50-55% der theoretischen Ausbeute erhalten werden. Die in den jeweiligen Mutterlaugen verbleibenden Produkte sind keiner weiteren Auftrennung mehr zugänglich. Auch durch Auswahl verschiedener Lösungsmittelsysteme konnte diese Reinigungsmethode nicht verbessert werden.

Es bestand daher die Aufgabe, ein Verfahren zur Reindarstellung von 2-Chlor-6-alkoxy-3-nitropyridinen zu finden, bei dem die durch Nitrieren von 2-Chlor-6-alkoxypyridinen erhaltenen Produkte in einem einfachen Verfahrensschritt unter möglichst geringen Ausbeuteverlusten in möglichst reiner Form erhalten werden.

Die Lösung der Aufgabe erfolgt durch ein Verfahren gemäss der Ansprüche 1-6.

Es wurde gefunden, dass zur Lösung dieser Aufgabe bereits das Verfahren zur Nitrierung der 2-Chlor-6-alkoxypyridine für Ausbeute, Reinheit und die Reinigungsmöglichkeit der nitrierten Produkte ausschlaggebend ist. Erfolgt die Nitrierung in der Art, wie sie üblicherweise bei der Nitrierung organischer Produkte durchgeführt wird, dass nämlich das Gemisch aus Schwefel- und Salpetersäure zum vorgelegten 2-Chlor-6-alkoxypyridin gegeben wird, so bilden sich als hauptsächliche Nebenprodukte 2-Chlor-6-alkoxy-5-nitropyridine. Diese Isomere können nur äusserst mühsam vom jeweiligen Hauptprodukt abgetrennt werden.

Überraschenderweise finden sich nach der erfindungsgemäss durchgeführten Nitrierung, bei der die 2-Chlor-6-alkoxypyridine portionsweise zum vorgelegten Gemisch aus Schwefel- und Salpetersäure gegeben werden, nahezu keine 2-Chlor-6-alkoxy-5-nitropyridine.

Weiterhin wurde gefunden, dass sämtliche Verunreinigungen der bei Nitrierung von 2-Chlor-6-alkoxypyridinen erhaltenen Rohprodukte in einfacher Weise abgetrennt werden können, wenn die Rohprodukte mit einer Lösung einer alkalisch wirkenden Verbindung in einem protischen Lösungsmittel bei Raumtemperatur bis mässig erhöhter Temperatur unter Rühren mehrere Stunden lang behandelt werden. Diese Behandlung kann entweder so durchgeführt werden, dass das jeweils isolierte, nitrierte Rohprodukt in der alkalischen Lösung mehrere Stunden lang digeriert, anschliessend abfiltriert, mit destilliertem Wasser gewaschen und getrocknet wird, oder so, dass das nitrierte Rohprodukt in einem wasserunlöslichen, inerten organischen Lösungsmittel wie Toluol oder Xylol gelöst wird, diese Lösung unter intensivem Vermischen mehrere Stunden lang mit einer wässerigen, alkalischen Lösung in Kontakt gebracht und danach abgetrennt wird. Das gereinigte 2-Chlor-6-alkoxy-3-nitropyridin wird durch Abdestillieren des Lösungsmittels isoliert. Diese Isolierung kann auch dadurch erfolgen, dass das gereinigte Produkt aus der Lösung, die unter Umständen schon eingeengt ist, mit einem anderen Lösungsmittel, wie z. B. Hexan ausgefällt wird.

Diese Verfahrensschritte ermöglichen es, 2-Chlor-6-alkoxy-3-nitropyridine einfach, in hoher Reinheit und guter Ausbeute herzustellen. Es ist ein weiterer Vorteil dieses Verfahrens, dass es kontinuierlich durchgeführt werden kann.

Dem Verfahren zugänglich sind alle 2-Chlor-6-alkoxypyridine mit einer geradkettigen oder verzweigten Alkylkette, vorzugsweise solche mit einer Alkylkette von 1 bis 4 C-Atomen wie:

2-Chlor-6-methoxypyridin
2-Chlor-6-äthoxypyridin
2-Chlor-6-propoxypyridin
2-Chlor-6-i.-propoxypyridin
2-Chlor-6-butoxypyridin
2-Chlor-6-i.-butoxypyridin

Sie werden bei Temperaturen im Bereich von 0-40° C in der Weise nitriert, dass sie zu einem vorgelegten Nitriergemisch zudosiert werden. Danach werden sie zweckmässigerweise vom sauren Reaktionsgemisch abgetrennt und mit einer alkalisch wirkenden Verbindung behandelt.

Es ist einleuchtend, dass die Behandlungsdauer umgekehrt proportional zur Alkalität der Lösung und zur Behandlungstemperatur ist. So ist bei Einsatz der stark alkalischen Alkoholate eine kurzzeitige Behandlung mit auf bis zu 0°C gekühlten Lösungen erfolgreich. Es wäre zwar auch möglich, mit gekühlten, wässerigen, alkalischen Lösungen zu digerieren, jedoch wäre dies aufgrund der langen Behandlungszeiten wirtschaftlich nicht sinnvoll. Andererseits sind bei Temperaturen oberhalb 50° C Abbaureaktionen der 2-Chlor-6-alkoxy-3-nitropyridine im alkalischen Medium zu erwarten, die umso leichter eintreten, je grösser die Alkalität des Mediums ist.

Protische Lösungsmittel sind solche Lösungsmittel, die Protonen freisetzen können, z. B. Wasser oder Alkohole. Alkalische Lösungen in protischen Lösungsmitteln erhält man durch Auflösen von alkalisch wirkenden Verbindungen in solchen protischen Lösungsmitteln. Derartige Verbindungen sind die Alkali- und Erdalkalioxide und -hydroxide sowie Ammoniumhydroxid, aber auch Salze, die durch Hydrolyse alkalisch reagieren, wie z. B. Alkalicarbonate oder Trialkaliphosphate, andererseits aber auch wasserlösliche, organische Aminoverbindungen wie z. B. Methyl-, Dimethyl-,

Trimethyl-, Äthylamin, Äthanolamin oder Piperidin.

Die Menge der alkalischen Lösung soll so bemessen sein, dass eine gut zu handhabende Aufschlämmung bzw. eine ausreichende Kontaktmöglichkeit der Reaktionspartner erzielt wird. Die eingesetzte Alkalimenge soll mindestens der erwarteten Menge an Verunreinigungen äquivalent sein. Andererseits ist es zweckmässig, mit einem Überschuss an Alkali zu arbeiten, um die Reinigung in möglichst kurzer Zeit quantitativ durchführen zu können.

Das erfindungsgemässe Verfahren wird durch die folgenden Beispiele weiter erläutert.

*Beispiel 1:*

143,6 g (1 mol) 2-Chlor-6-methoxypyridin werden in einer Stunde zu einer auf 0°C gekühlten und gerührten Mischung aus 1600 ml konz. Schwefelsäure und 800 ml rauchender salpetersäure getropft. Anschliessend lässt man in 3 h die Temperatur auf 20°C steigen und rührt dann bei gleicher Temepratur noch 3 weitere Stunden nach. Danach wird die Mischung auf 5 kg gemahlenes Eis gegossen, das ausgefallene Produkt abgesaugt und mit viel Wasser säurefrei gewaschen. Nach dem Trocknen werden 151-6 g (80-3% d. Th.) in Form von gelben Kristallen in 84-5%iger Reinheit vom Schmelzpunkt 67-9°C erhalten.

*Beispiel 2:*

Eine Mischung aus 3300 g gemäss Beispiel 1 erhaltenem, verunreinigtem fein pulverisiertem 2-Chlor-6-methoxy-3-nitropyridin, 6600 ml Wasser und 910 g Kaliumcarbonat wird 2 h bei 40°C gerührt. Anschliessend wird das Produkt abgesaugt, mit 4000 ml Wasser gewaschen und bei 40-45°C getrocknet. Es werden 2772-2805 g 2-Chlor-6-methoxy-3-nitropyridin mit einem Schmelzpunkt 79°C und einer Reinheit von 99-100% erhalten.

*Beispiel 3:*

157,6 g (1 mol) 2-Chlor-6-äthoxypyridin werden innerhalb von 45 min unter Rühren zu einer Mischung aus 190 ml konzentrierter Schwefelsäure, 190 ml 24%igem. Oleum und 100 ml 97%iger. Salpertersäure zudosiert. Das Reaktionsgemisch wird durch Kühlen im Temperaturbereich von 20 bis 40°C gehalten. Danach lässt man unter Rühren noch weitere 5 h nachreagieren, wobei sich die Temperatur der Raumtemperatur angleicht. Anschliessend wird das Reaktionsgemisch auf 0°C abgekühlt, über eine Glasfritte abgesaugt, das erhaltene feste Rohprodukt in 250 ml zusammen mit 100 g Kaliumcarbonat in 250 ml Wasser suspendiert und 3 h lang bei 30°C gerührt.

Danach wird das Produkt abgesaugt, mit Wasser gewaschen und bei 40°C getrocknet.

Ausbeute: 192 g (93% d. Th.) 2-Chlor-6-äthoxy-3-nitropyridin in einer Reinheit von 98%.

*Beispiel 4:*

Eine Lösung von 500 g 84-85%igem, gemäss Beispiel 1 erhaltenem 2-Chlor-6-methoxy-3-nitropyridin in 1500 ml Toluol wird 12 h bei 40°C mit 500 ml 24%igem Ammoniakwasser intensiv gerührt. Anschliessend wird der Niederschlag abgesaugt, die Phasen getrennt, von der Toluolschicht im Vakuum bei max. 90°C Sumpftemperatur das Lösungsmittel abdestilliert und zu dem flüssigen Destillationsrückstand Heptan (∼ 1000 ml) eingerührt. Nach dem Abkühlen werden die ausgefallenen Kristalle abgesaugt und bei 40-45°C getrocknet. Es werden 345 g 2-Chlor-6-methoxy-3-nitropyridin in 99-100%iger Reinheit mit einem Schmelzpunkt 79°C erhalten. Durch Einengen der organischen Mutterlauge bis fast zur Trockne und Durchrühren mit Heptan werden weitere 47 g vom Schmelzpunkt 78-79°C isoliert. Die Gesamtausbeute beträgt dann 92% d. Th.

*Beispiel 5:*

Eine Lösung von 500 g 84-85%igem, gemäss Beispiel 1 erhaltenem 2-Chlor-6-methoxy-3-nitropyridin in 1500 ml Toluol wird mit einer Lösung von 124 g Monomethylamin in 600 ml 50%igem Methanol 10 h bis 0-5°C gerührt. Anschliessend wird der Niederschlag abfiltriert und die Phasen getrennt. Die Isolierung des reinen 2-Chlor-6-methoxy-3-nitropyridins erfolgt wie im Beispiel 4 beschrieben. Ausbeute 90-3% d. Th.

## Patentansprüche

1. Verfahren zur Reindarstellung von 2-Chlor-6-alkoxy-3-nitropyridinen durch Nitrierung von 2-Chlor-6-alkoxypyridinen mit Salpetersäure und Isolierung der nitrierten Produkte, dadurch gekennzeichnet, dass die Nitrierung in der Weise erfolgt, dass die 2-Chlor-6-alkoxypyridine im Temperaturbereich von 0-40°C portionsweise zu einem vorgelegten Gemisch aus konzentrierter Schwefelsäure und konzentrierter Salpetersäure zudosiert werden und die dabei erhaltenen, noch verunreinigten 2-Chlor-6-alkoxy-3-nitropyridine mit einer Lösung einer alkalisch wirkenden Verbindung in einem protischen Lösungsmittel behandelt und anschliessend rein isoliert werden.

2. Verfahren nach Anspruch 1, dadurch gekennzeichnet, dass das verunreinigte Produkt in einer alkalischen wässerigen Lösung digeriert und anschliessend abfiltriert, gewaschen und getrocknet wird.

3. Verfahren nach Anspruch 1, dadurch gekennzeichnet, dass das verunreinigte Produkt in einem inerten, wasserunlöslichen Lösungsmittel gelöst, diese Lösung unter intensivem Vermischen mit der wässerigen, alkalischen Lösung in Kontakt gebracht und danach abgetrennt wird, wonach das gereinigte Produkt in an sich bekannter Weise isoliert wird.

4. Verfahren nach Ansprüchen 1-3, dadurch gekennzeichnet, dass die Behandlung des verunreinigten Produkts bei einer Temperatur im Bereich von 0-50°C durchgeführt wird.

5. Verfahren nach Ansprüchen 1-3, dadurch gekennzeichnet, dass als alkalische, wässerige Lösung eine Lösung eines Alkalicarbonats verwendet wird.

6. Verfahren nach Ansprüchen 1-3, dadurch gekennzeichnet, dass als alkalische wässerige Lösung Ammoniakwasser verwendet wird.


## Claims

1. A process for the preparation of pure 2-chloro-6-alkoxy-3-nitro-pyridines by nitration of 2-chloro-6-alkoxy-pyridine with nitric acid and isolation of the nitrated products, characterized in that the nitration is made by adding in portions the 2-chloro-6-alkoxy-pyridines to a mixture of concentrated sulfuric acid and concentrated nitric acid at a temperature in the range of 0 to 40°C and that the resulting, still impure 2-chloro-6-alkoxy-3-nitro-pyridines are treated with a solution of an alkaline reacting compound in a protic solvent and recovered in substantially pure form.

2. The process of Claim 1 wherein the impure product is digested with an aqueous alkaline solution and the pure product is then recovered by filtration, washing and drying.

3. The process of Claim 1 wherein the impure product is dissolved in an inert, water-immiscible organic solvent, the resulting organic solution is contacted with vigorous agitation with an aqueous alkaline solution, then separated and then the pure product is recovered by known manner.

4. The process of Claims 1-3 wherein the treatment of the impure product is effected at 0 to 50°C.

5. The process of Claims 1-3 wherein the alkaline aqueous solution is a solution of an alkali metal carbonate.

6. The process of Claims 1-3 wherein the alkaline aqueous solution is aqueous ammonium hydroxide.


## Revendications

1. Procédé de préparation sous une forme pure de 2-chloro-6-alcoxy-3-nitropyridines par nitration de 2-chloro-6-alcoxypyridines avec de l'acide nitrique et isolement des produits nitrés, caractérisé par le fait que la nitration est effectuée de telle manière que les 2-chloro-6-alcoxypyridines sont ajoutées, dans un domaine de température de 0 à 40°C, par portions à un mélange prêt d'acide sulfurique concentré et d'acide nitrique concentré et que les 2-chloro-6-alcoxy-3-nitropyridines obtenues sous une forme encore non purifiée sont traitées avec une solution d'un composé à activité alcaline dans un solvant protique et qu'on les isole ensuite dans un état pur.

2. Procédé selon la revendication 1, caractérisé par le fait que le produit non purifié est digéré dans une solution aqueuse alcaline et ensuite filtré, lavé et séché.

3. Procédé selon la revendication 1, caractérisé par le fait que le produit non purifié est dissous dans un solvant inerte, insoluble dans l'eau, cette solution est mise en contact avec mélange intensif avec une solution aqueuse alcaline et séparée ensuite de celle-ci, le produit pur étant alors isolé de manière connue.

4. Procédé selon les revendications 1 à 3, caractérisé par le fait que le traitement du produit non purifié est réalisé à une température dans un domaine de 0 à 50°C.

5. Procédé selon les revendications 1 à 3, caractérisé par le fait qu'on utilise comme solution aqueuse alcaline une solution d'un carbonate alcalin.

6. Procédé selon les revendications 1 à 3, caractérisé par le fait qu'on utilise comme solution aqueuse alcaline de l'ammoniaque pure.